(19) Europäisches Patentamt
European Patent Office
Office européen des brevets

(11) EP 0 887 330 B1

(12) **EUROPÄISCHE PATENTSCHRIFT**

(45) Veröffentlichungstag und Bekanntmachung des Hinweises auf die Patenterteilung:
**25.09.2002 Patentblatt 2002/39**

(51) Int Cl.[7]: **C07C 41/08**, C01B 33/20, C07C 41/54, B01J 23/06, C07C 43/16, C07C 43/303

(21) Anmeldenummer: **98110001.9**

(22) Anmeldetag: **02.06.1998**

(54) **Verfahren zur Addition von Hydroxylgruppen enthaltenden Verbindungen an Alkine oder Allene**

Process for the addition of hydroxyl group-containing compounds to alkynes or allenes

Procédé pour l'addition de composés contenant un groupe hydroxyle sur les alcynes ou les allènes

(84) Benannte Vertragsstaaten:
**CH DE FR GB IT LI**

(30) Priorität: **23.06.1997 DE 19726670**

(43) Veröffentlichungstag der Anmeldung:
**30.12.1998 Patentblatt 1998/53**

(60) Teilanmeldung:
**00116291.6 / 1 050 510**
**00116292.4 / 1 050 523**

(73) Patentinhaber: **BASF AKTIENGESELLSCHAFT**
**67056 Ludwigshafen (DE)**

(72) Erfinder:
- **Teles, Joaquim Henrique, Dr.**
  **67122 Altrip (DE)**
- **Rieber, Norbert, Dr.**
  **68259 Mannheim (DE)**
- **Breuer, Klaus, Dr.**
  **67122 Altrip (DE)**
- **Demuth, Dirk, Dr.**
  **68161 Mannheim (DE)**
- **Hibst, Hartmut, Prof.Dr.**
  **69198 Schriesheim (DE)**
- **Hagemeyer, Alfred, Dr.**
  **48431 Rheine (DE)**

(74) Vertreter: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:
**EP-A- 0 165 647** **US-A- 2 521 113**

- **CHEMICAL ABSTRACTS, vol. 112, no. 7, 12. Februar 1990 Columbus, Ohio, US; abstract no. 54986a, O. N. TEMKIN: "A catalyst for the manufacture of methyl and ethyl isopropenyl ethers" Seite 687; Spalte 1; XP002080414 & DD 267 629 A**
- **CHEMICAL ABSTRACTS, vol. 112, no. 7, 12. Februar 1990 Columbus, Ohio, US; abstract no. 54987b, O. N. TEMKIN: "A catalyst for the manufacture of methyl and ethylisopropenyl ether" Seite 687; Spalte 1; XP002080415 & DD 265 289 A**
- **CHEMICAL ABSTRACTS, vol. 69, no. 26, 23. Dezember 1968 Columbus, Ohio, US; abstract no. 108210g, L. S. PAKHOIMOVA: "Cadmium silicate and zinc silicate catalysts" Seite 10156; Spalte 1; XP002080416 & SU 220 957 A**
- **CHEMICAL ABSTRACTS, vol. 119, no. 22, 29. November 1993 Columbus, Ohio, US; abstract no. 231586n, H. NAGATA: "Analytical study of the formation of hemimorphite. I. Analysis of the crystallization process by the co-precipitation method" Seite 380; Spalte 2; XP002080417 & ZAIRYO TO KANKYO, Bd. 42, Nr. 4, 1993, Seiten 225-233,**
- **CHEMICAL ABSTRACTS, vol. 72, no. 6, 9. Februar 1970 Columbus, Ohio, US; abstract no. 27871h, A. G. MERKULOV: "Low-temperature synthesis of zinc silicates" Seite 519; Spalte 1; XP002080675 & IZV. SIB. OTD. AKAD. NAUK SSSR, SER. KHIM. NAUK, Nr. 4, 1969, Seiten 70-74,**
- **CHEMICAL ABSTRACTS, vol. 102, no. 24, 17. Juni 1985 Columbus, Ohio, US; abstract no. 206083n, "Zinc silicate" Seite 128; Spalte 1; XP002080676 & JP 60 011219 A (MITSUBISHI ELECTRIC)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 887 330 B1

## Beschreibung

Die Erfindung betrifft ein Verfahren zur Addition von Hydroxylgruppen enthaltenden Verbindungen an Alkine oder Allene unter Bildung von Aldehyden und Ketonen oder deren Derivaten in Form von Enolethern oder Acetalen bzw. Ketalen in Gegenwart eines röntgenamorphen Zinksilikats-Katalysators. Ferner betrifft die Erfindung ein neues Verfahren zur Herstellung eines solchen röntgenamorphen Zinksilikats und den so erhaltenen Katalysator.

Die Addition von Hydroxylgruppen enthaltenden Verbindungen an Alkine bzw. Allene wird fast ausnahmsweise mit homogen gelösten Katalysatoren durchgeführt, z.B. mit Säuren, Basen und Übergangsmetallkomplexen (vgl. Houben-Weyl, Methoden der organischen Chemie, Bd 6/3, S. 233, S. 90, Bd. 5/2a, S. 738, Bd. 6/1d, S. 136 und Bd. 7/a, S. 816).

Die Säurekatalyse ist meistens auf die Addition an aktivierte, elektronenreiche Alkine (wie Acetylenether, $R-C{\equiv}C-OR'$,Acetylenthioether, $R-C{\equiv}C-SR'$ und Acetylenamine, $R-C{\equiv}C-NR'_2$) beschränkt.

Basenkatalysiert (in Gegenwart von KOH oder Alkoholat) können Alkohole in der Flüssigphase auch an nicht aktivierte Alkine addiert werden. Dies ist die gebräuchlichste Methode; allerdings bedarf die Reaktion hoher Temperaturen und Drücke und die Raumzeitausbeute ist relativ gering. Typischerweise benötigt man für die basenkatalysierte Vinylierung eines Alkoholes zwischen 6 und 10 h Verweilzeit bei ca. 160°C und 18 bis 20 bar Druck.

Die Addition kann auch durch Übergangsmetallkomplexe in der Flüssigphase katalysiert werden. Für die Addition von Alkoholen eignen sich insbesondere Quecksilber(II)- oder Gold(I)-Salze, während für die Addition von Carbonsäuren und Phenole Zink- und Cadmiumsalze bevorzugt werden.

Die Addition von Carbonsäuren (insbesondere Essigsäure und Propionsäure) an Acetylen kann auch in der Gasphase in Gegenwart entsprechender Zinkcarboxylate (auch basischer Zinkcarboxylate gemäß CH 239752) auf oberflächenreichen Trägern als Katalysatoren durchgeführt werden.

In der US 2,521,113 wird ein Verfahren zur Herstellung von Carbonsäurevinylestern beschrieben, wobei eine gasförmige Mischung einer Carbonsäure und Acetylen bei erhöhter Temperatur über einen Katalysator geführt werden, der im wesentlichen aus einem Zink- oder Cadmiumsilikat oder - polysilikat besteht und das Acetylen im Überschuss eingesetzt wird.

In der EP 0 165 647 wird ein Verfahren zur Herstellung eines synthetischen kristallinen Zinksilikats beschrieben, wobei ein mit Säure behandeltes Produkt eines Tonminerals und ein Zinkoxid oder -hydroxid unter hydrothermalen Bedingungen miteinander umgesetzt werden.

H. Nagata (Chemical Abstracts, Vol. 119, No.22, Abstract No. 231586n) beschreibt die Herstellung von Hemimorphit, wobei kolloidales Zn $(OH_2)$ mit einer Lösung von $Na_2$ $SiO_3$ x 9 $H_2O$ kopräzipitiert werden und der Niederschlag bei 85° Celsius für 0 bis 120 Tage gealtert wird.

JP 60-11219 (Chemical Abstracts, Vol. 102, No. 24, 1985, Abstract No. 206083n beschreibt die Herstellung von Zinksilikaten die 57,9% Zn und 42,1% Si enthalten. Um eine Kopräzipitation von Zn $(OH)_2$ zu vermeiden, wird $NH_3$ oder ein organisches Amin einer Lösing von Zn $(NO_3)_2$ x 6 $H_2O$ und $Na_2$ $SiO_3$ x 9 $H_2O$ beigegeben.

Merkulov et. al. (Chemical Abstracts, Vol. 72, No. 61970, Abstract No. 27875 1 h beschreibt die Herstellung kristalliner Zinksilikate bei Temperaturen von 90-100°C. Bei einem pH von 10-12 wird Hemimorphit gebildet, bei einem pH von 6,5 - 8,5 Willemit und Zinksilit bei einem pH von 5-6.

Schließlich ist auch bereits die Addition von Methanol an Propin bzw. Propadien in der Gasphase in Gegenwart von Zinkoxid auf Aktivkohle oder Silikagel in DD 265 289 bzw. von Zinknitrat auf Aktivkohle oder Silikagel in DD 267 629 beschrieben.

Alle diese Verfahren des Standes der Technik haben Nachteile. Sie haben entweder nur ein beschränktes Anwendungsgebiet oder benötigen, wie die basenkatalysierte Addition, hohe Drücke und Temperaturen, was zu Sicherheitsproblemen führen kann, oder sie haben nur eine geringe Raumzeitausbeute. Homogen gelöste Übergangsmetallkatalysatoren sind oft nach einer geringen Anzahl von Zyklen desaktiviert und sind außerdem schwierig zurückzuführen. Heterogene Katalysatoren für die Addition an Alkine bzw. Allene sind bisher nur selten beschrieben worden. Zink- bzw. Cadmiumcarboxylate auf Aktivkohle katalysieren lediglich die Addition von Carbonsäuren (z.B. Essigsäure oder Propionsäure) an Acetylen. Der o.g. Katalysator auf der Basis von Zinkoxid auf Aktivkohle oder Silicagel (DD 265 289) ist zwar in der Lage die Addition von Alkoholen (Methanol oder Ethanol) an Propin bzw. Propadien mit guter Selektivität (90 bis 96 %) zu katalysieren, die katalytische Aktivität ist aber relativ gering und die benötigten Reaktionstemperaturen und Kontaktzeiten sind hoch. Zinknitrat auf Aktivkohle oder Silicagel ist bei gleicher Temperatur ungefähr eine Größenordnung weniger aktiv als das Zinkoxid und die Selektivität ist viel geringer (max. 70 %).

Es bestand daher die Aufgabe, einen möglichst bei niedrigen Temperaturen aktiven und selektiven heterogenen Katalysator für die Addition von Hydroxylgruppen enthaltende Verbindungen an Alkine, Allene oder Gemische davon vorzuschlagen.

Diese Aufgabe wurde erfindungsgemäß gelöst mit einem Verfahren zur Herstellung von Verbindungen der Formeln I bzw. II

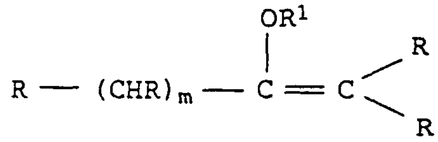

I

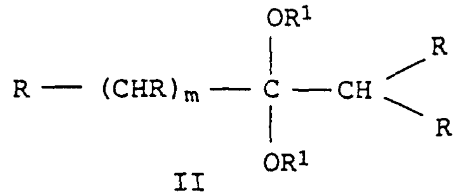

II

in denen $R^1$ Wasserstoff oder einen aliphatischen, cycloaliphatischen, araliphatischen, aromatischen oder heterocyclischen Rest oder einen Acylrest bedeutet, wobei diese Reste weitere Substituenten, die nicht mit Acetylenen oder Allenen reagieren, tragen können, die Reste R unabhängig voneinander für Wasserstoff, oder aliphatische, cycloaliphatische, araliphatische, aromatische oder heterocyclische Reste stehen, die unter Bildung eines Ringes miteinander verbunden sein können, und m für 0 oder 1 steht, durch Addition von Verbindungen der Formel III

$$R^1OH \qquad \text{III}$$

an Acetylene oder Allene der Formeln IV bzw. V

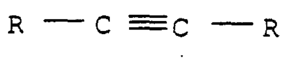

IV

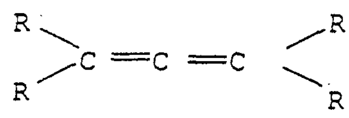

V

wobei $R^1$ und R die oben angegebenen Bedeutungen haben, in der Gasphase bei erhöhter Temperatur in Gegenwart eines ein Zinksilikat enthaltenden Katalysators, bei dem man einen Katalysator verwendet, der als aktiven Bestandteil ein durch Ausfällung in wäßriger Lösung aus einer Siliziumverbindung, insbesondere Erdalkali- oder Alkalisilikat, und einer Zinkverbindung, insbesondere einem Zinksalz, erhältliches Zinksilikat enthält oder aus diesem besteht, wobei das Zinksilikat

a) ein im wesentlichen röntgenamorphes Zinksilikat der Formel VI

$$Zn_aSi_cO_{a+2c-0,5e}(OH)_e.fH_2O \qquad \text{VI,}$$

in der e die Werte 0 bis zur Summe aus $2_a + 4_c$ bedeutet und das Verhältnis a:c 1 bis 3,5, bevorzugt 1,5 bis 2,8, besonders bevorzugt 1,9 bis 2,3 und insbesondere 2 bis 2,1, und das Verhältnis f:a 0 bis 200, bevorzugt 0 bis 2,5 und besonders bevorzugt 0 bis 0,25 beträgt und/oder

b) daß es durch Ausfällung aus wäßriger Lösung aus einem Alkalisilikat und einem Zinksalz bei einem ph-Wert von 4 bis 9,5 erhalten worden ist.

Der erfindungsgemäß zu verwendende amorphe Katalysator (a) läßt sich auch physikalisch charakterisieren. Er weist bei Anwendung von Cu-$K_{a-1}$-Strahlung (1 = 1,5406 Å) im Pulverröntgenbeugungsdiagramm zwei breite Banden bei 2q = 31 ± 5° und bei 2q = 61 ± 7° auf.

Die erfindungsgemäß zu verwendenden Zinksilikate können mit bis zu 80, vorzugsweise 50 und insbesondere bis zu 20 Molprozent noch mit weiteren Metallen dotiert sein ausgewählt aus der Gruppe (A) bestehend aus Beryllium, Magnesium, Calzium, Strontium, Barium, Mangan, Eisen, Kobalt, Nickel, Kupfer, Cadmium und Quecksilber und der Gruppe (B), bestehend aus Titan, Zirkonium, Hafnium, Germanium, Zinn und Blei, wobei die Elemente der Gruppe (A) teilweise Zink und die Elemente der Gruppe (B) teilweise Silizium in der Formel VI ersetzen. Im Falle von Cadmium kann Zn auch vollständig durch Cadmium ersetzt sein. Doch bieten Cadmiumsilikate wegen der Toxizität des Cadmiums keine Vorteile.

Im Falle des Verhältnisses von Zink zu Silizium von ungefähr 2 entspricht der Katalysator der Formel VI der

Zusammensetzung des (kristallinen) Hemimorphits, in dem die erfindungsgemäßen röntgenamorphen Zinksilikate der Formel VI durch längeres Erhitzen in wäßriger Suspension übergehen können.

Die Herstellung von Hemimorphit ist aus der Literatur bekannt. Sie kann unter Normalbedingungen oder hydrothermalen Bedingungen erfolgen.

A) Hemimorphit-Katalysator

a) Herstellung unter Normalbedingungen

A.G. Merkulov u. B. S. Khristoforov, (Tr. Soveshch. Eksp. Tekh. Mineral. Petrogr., $8^{th}$ (1971), Meeting Date 1968, 322-8; ditor(s): V. V. Lapin; Publisher: "Nauka", Moscow, USSR) beschreiben die Herstellung verschiedener Zn-Silikate durch eine Reaktion von verschiedenen Zinksalzen (Carbonat, Sulfat, Chlorid, Acetat, Oxid) mit Na-Silikat und Natronlauge in wäßriger Lösung bei Temperaturen von 90 - 100°C und bei Normaldruck. Dabei bilden sich in Abhängigkeit vom eingestellten pH-Wert unterschiedliche Zinksilikate. So entsteht reiner Sauconit mit der Zusammensetzung $Zn_3Si_4O_{10}(OH)_2 \cdot n\ H_2O$ bei einem End-pH-Wert von 5 - 6. Reiner Willemit ($\alpha$-$Zn_2SiO_4$) fällt im pH-Bereich von 6,5 - 8,5 an. Reiner Hemimorphit ($Zn_4Si_2O_7(OH)_2 \cdot H_2O$) kristallisiert dagegen nur im schwach alkalischen Medium bei pH-Werten von größer 10 aus.

In einer anderen Arbeit der genannten Autoren (A. G. Merkulov u. B. S. Khristoforov, Izv. Sib. Otd. Akad. Nauk SSSR, Ser. Khim. Nauk (1969), (4), 70 - 4) wird angegeben, daß reiner Hemimorphit bei der Umsetzung von Zinksalzen mit Natriumsilikat und Natronlauge bei 90 - 100°C und Atmosphärendruck in wäßriger Lösung nur in einem pH-Bereich von 10 - 12 gebildet wird.

Ferner konnten T. Baird, A. G. Cairns Smith und D. S. Snell (Reactivity of Solids, Proc. Int. Symp., 8th (1977), Göteborg, Meeting Date 1976, 337 - 42; Editor(s): J. Wood, O. Lindqvist u. C. Helgesson; Publisher: Plenum Press, New York, N. Y.) große Kristalle von Hemimorphit durch Umsetzung von $Zn(OH)_2$ mit Kieselsäure und LiOH in wäßriger Lösung bei einem pH von 10 herstellen.

Schließlich wurde von H. Nagata, M. Matsunage u. K. Hosokawa (Zairyo-to-Kankyo (1993) 42, 225 - 233) Hemimorphit hergestellt, indem wäßrige Zinksulfat-Lösung mit Natronlauge und wäßriger Natriumsilikat-Lösung bei einem pH von 13 umgesetzt wurde, der erhaltene Niederschlag abgetrennt, ausgewaschen und bei 85°C mindestens 24 Stunden lang gealtert wurde.

b) Hydrothermale Herstellung

Gemäß EP 165 647 kann Hemimorphit aus einem säurebehandelten Tonmineral und Zinkoxid bzw. Zinkhydroxid unter hydrothermalen Bedingungen (170°C, 5 h) hergestellt werden. Die Säurevorbehandlung des Tones ist jedoch sehr aufwendig und deshalb ist dieses Verfahren nachteilig.

Gemäß D. M. Roy u. F. A. Mumpton (Econ. Geol. (1956) 51, 432 - 443) kann Hemimorphit ferner durch hydrothermale Umsetzung von Mischungen aus ZnO und $SiO_2$ bei 175 - 200°C gewonnen werden (Zusammensetzung : 3 ZnO + 2 $SiO_2$). Das erhaltene Produkt enthält überwiegend Hemimorphit, ist aber durch Sauconit ($Zn_3Si_4O_{10}(OH)_2 \cdot 4H_2O$) verunreinigt.

Schließlich beschreiben P. Taylor u. D. G. Owen, (Polyhedron (1984) 3(2) 151-155) die hydrothermale Synthese von Hemimorphit durch Umsetzung von ZnO mit $SiO_2$ in wäßriger Lösung bei 150°C. Zur Herstellung von Produkten mit einem hohen Hemimorphit-Anteil waren jedoch lange Reaktionszeiten von mindestens 4 Tagen erforderlich.

Obgleich Hemimorphit-Produkte, die nach den oben beschriebenen bekannten Methoden erhalten wurden, sich als Katalysator für die erfindungsgemäße Addition sehr gut eignen, ergab sich, daß es wünschenswert war, deren Eigenschaften noch zu verbessern und eine Herstellungsmethode vorzuschlagen, mit deren Hilfe sich Katalysatoren mit reproduzierbar gutem Eigenschaftsprofil herstellen lassen.

Demgemäß wurde eine neue Herstellungsmethode sowohl unter Normaldruck als auch unter hydrothemalen Bedingungen gefunden, bei der man ein Alkali- oder Erdalkalisilikat, vorzugsweise Natriumsilikat mit einem Zinksalz, insbesondere Zinknitrat, und einer Base wie Alkali- oder Erdalkalihydroxid, insbesondere Natriumhydroxid in wäßriger Lösung bei pH-Werten von 4 bis 9,5 vorzugsweise 5,5 bis 8 und insbesondere in einem pH-Bereich um den Neutralpunkt, z.B. bei pH 6 bis 7,5, im Falle von Normaldruck bei Temperaturen von 50 bis 100°C, insbesondere 70 bis 100°C und im Falle von hydrothermalen Bedingungen bei Temperaturen von 100 bis 250°C, bevorzugt im Bereich von 100 bis 200°C umgesetzt.

Nach der neuen Herstellungsmethode kann reiner Hemimorphit mit einem Zn/Si-Verhältnis von 2 synthetisiert werden. Es sind aber auch Hemimorphit-Präparate mit bis zu 25 % Unter- oder Überschuß an Zink, entsprechend einem Atomverhältnis Zn:Si von 1,6 bis 2,5 zugänglich. Als Katalysatoren werden Hemimorphite bevorzugt, die 0 bis 20 % Zinküberschuß enthalten. Besonders bevorzugt sind Hemimorphite, die 0 bis 10 % Zinküberschuß enthalten.

Die Hemimorphit-Produkte fallen bei der Synthese als weißer kristalliner Niederschlag in Form einer

wäßrigen Suspension an und müssen durch geeignete Maßnahmen, z.B. Filtration oder Zentrifugieren, von der wäßrigen Lösung getrennt werden. Im Falle der Filtration wird der erhaltene Filterkuchen daraufhin ausgewaschen, um es von löslichen Salzen zu befreien, und anschließend getrocknet. Die Trocknung kann bei Temperaturen bis 600°C erfolgen, wobei der bevorzugte Temperaturbereich 90 bis 450°C umfasst. Thermogravimetrische Untersuchungen haben gezeigt, daß der auskristallisierte Hemimorphit der Zusammensetzung $Zn_4Si_2O_7(OH)_2 \cdot H_2O$ im Temperaturbereich von etwa 100 bis 200°C unter Beibehaltung der Hemimorphit-Struktur zunehmende Anteile seines Kristallwassers verliert, wobei Hemimorphit-Präparate der Zusammensetzung $Zn_4Si_2O_7(OH)_2 \cdot xH_2O$ mit x kleiner als 1 resultieren, wobei x mit steigender Temperatur abnimmt. Trocknet man in einem höheren Temperaturbereich von etwa 200 bis 600°C, so werden zusätzlich, ebenfalls unter Beibehaltung der Hemimorphit-Struktur, die im Hemimorphit enthaltenen $OH^-$-Ionen in $O^{2-}$-Ionen und abgespaltenes $H_2O$ umgewandelt ($2\ OH^- \rightarrow H_2O + O^{2-}$), wobei Hemimorphit-Präparate der Zusammensetzung $Zn_4Si_2O_7(OH)_{2-2y}O_y$ mit y=0 bis 1 resultieren, wobei y mit steigender Temperatur zunimmt.

Die nach der Trocknung bei Temperaturen bis 600°C, bevorzugt bei 90 bis 450°C erhaltenen Hemimorphit-Präparate der Zusammensetzung $Zn_4Si_2O_7(OH)_{2-2y}O_y \cdot xH_2O$, wobei x und y Werte von 0 bis 1 umfasst, werden anschließend üblicherweise mit den gebräuchlichen Formgebungsverfahren, z B. Tablettieren, Verstrangen oder als Schalenkatalysatoren auf Steatitkugeln zu katalytischen Formkörpern verarbeitet. Einzelheiten werden in den Beispielen beschrieben.

B) Röntgenamorpher Zinksilikat-Katalysator

Es wurde nun gefunden, daß man im wesentlichen unter gleichen Herstellungsbedingungen aber kürzerer Umsetzungszeit als Vorstufe zur Herstellung eines kristallinen Hemimorphits ein röntgenamorphes Produkt mit verbesserten katalytischen Eigenschaften erhält.

Dazu wird erfindungsgemäß z.B. eine wäßrige Suspension eines Alkali- oder Erdalkalisilikats mit einer wäßrigen Lösung eines Zinksalzes bei

a) Temperaturen von 20°C, bevorzugt 50°C bis zum Siedepunkt der sich ergebenden wäßrigen Suspension bei

b) einem pH-Wert von 4 bis 9,5, bevorzugt bei einem pH-Wert in der Nähe des Neutralpunktes,

c) und solchen Mengenverhältnissen von Alkalisilikat und Zinksalz umgesetzt, daß die Bedingungen der Formel VI erfüllt werden und

d) eine solche Verweilzeit eingehalten, daß noch nicht in erheblichem Maße Kristallisation des Zinksilikats eintritt.

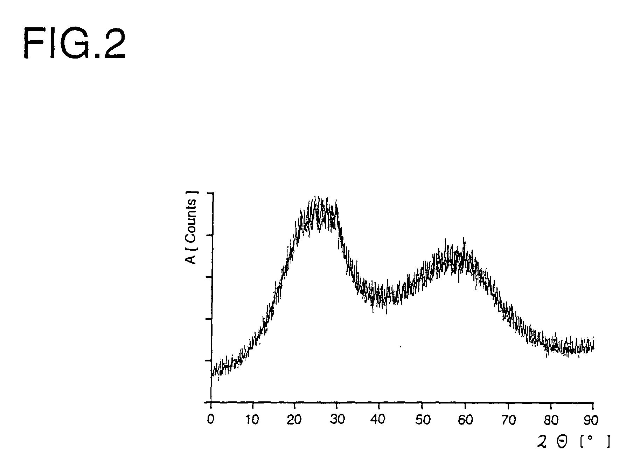

Das so erhältliche im wesentlichen röntgenamorphe Zinksilikat enthält $Zn^{2+}$-, $Si^{4+}$ und $O^{2-}$Ionen; darüber hinaus kann die Verbindung OH-Ionen und Hydratwasser enthalten. Das Zn/Si-Verhältnis beträgt 0,3 bis 5, bevorzugt 1 bis 2,7, besonders bevorzugt 2 bis 2,3 und ganz besonders bevorzugt 2. In letzterem Fall weist das röntgenamorphe Zinksilikat damit das Zn/Si-Verhältnis des kristallinen Hemimorhpits ($Zn_4Si_2O_7(OH)_2 \cdot H_2O$) auf. Das unter Anwendung von Cu-$K\alpha_1$-Strahlung ($\lambda$ = 1,5406 Å) erhaltene Pulver-Röntgenbeugungsdiagramm des erfindungsgemäßen röntgenamorphen Zinksilikats ist in Fig. 2 wiedergegeben, wobei die Intesität A der gebeugten Röntgenstrahlung als Funktion des zweifachen Beugungswinkels ($2\theta$) aufgetragen ist. Das Pulver-Röntgenbeugungsdiagramm des erfindungsgemäßen röntgenamorphen Zinksilikats weist im $2\theta$-Bereich von 10° bis 90° sehr breite Intensitätsmaxima bei $2\theta = 31° \pm 5°$ und bei $2\theta = 61° \pm 7°$ auf. In Fig. 2 sind über die genannten breiten Beugungsreflexe des erfindungsgemäßen röntgenamorphen Zinksilikats hinaus weitere schärfere Linien zu erkennen, die der Bildung geringer Mengen an kristallinem ZnO zugeordnet werden können (Karteikarte 5-0664 der JCPDS-ICDD-Kartei (1995)). Daneben können auch kleine Mengen an $Zn_5(NO_3)_2(OH)_8 \cdot 2\ H_2O$ auftreten (Karteikarte 24-1460 der JCPDS-ICDD-Kartei (1995)).

Das neue röntgenamorphe Zinksilikat fällt bei der Herstellung als Pulver an. Dieses Pulver kann als solches für die katalytische Umsetzung eingesetzt werden (z.B. in einem Wirbelbettreaktor) oder nach Verformung (z.B. Verstrangung, Tablettierung, etc., evtl. auch unter Zusatz von Hilfsstoffen) in einer für einen Festbettreaktor geeigneten Form.

Vor dem Einsatz kann der Katalysator bei Temperaturen zwischen 80°C und 750°C kalziniert werden. Bevorzugt wird der Katalysator zwischen 120°C und 500°C kalziniert. Besonders bevorzugt ist die Kalzinierung zwischen 200° und 400°C an der Luft. Zur Erhöhung des Porenvolumens können bei der Formgebung z.B. beim Tablettieren oder Verstrangen auch Porenbildner zugesetzt werden (z.B. Superabsorber wie Lutexal P® (Firma BASF AG) oder Walocel® (Methylcellulose/Kunstharz-Kombination, Firma Wolff, Walsrode AG)).

Als Edukte für die erfindungsgemäße Umsetzung kommen beliebige Alkine oder Allene oder Gemische davon in Betracht. In der Regel wird man jedoch technisch leicht zugängliche Acetylene und Allene mit 2 bis 8 C-Atomen, bzw. 3 bis 8 C-Atomen verwenden. Besonders bevorzugt sind Propin und Allen und insbesondere Kohlenwasserstoffströme, die diese enthalten.

Die Hydroxylgruppen enthaltende Verbindung $R^1OH$ kann Wasser, ein beliebiger Alkohol, ein Phenol oder eine Carbonsäure sein. Im allgemeinen kommen vor allem Alkohole, besonders Alkanole mit 1 bis 16 C-Atomen, einkernige Phenole und niedermolekulare Carbonsäuren, z.B. mit 1 bis 16 C-Atomen, in Betracht. Besonders bevorzugt werden niedere Alkohole und insbesondere Methanol verwendet.

Die Addition der Hydroxylgruppen enthaltenden Verbindungen erfolgt in Gegenwart des heterogen vorliegenden Katalysators in der Gasphase entweder über einem Festbett oder in einem Wirbelbett bei Temperaturen von 50 bis 400°C, vorzugsweise 100 bis 250°C und besonders bevorzugt 120 bis 200°C und Drücken von 0,1 bis 100 bar, insbesondere 0,8 bis 20 bar (alle Drücke bezogen auf Summe der Partialdrücke der Edukte).

Gegebenenfalls kann das Reaktionsgemisch aus Gründen der Betriebssicherheit und der besseren Wärmeabfuhr mit Inertgasen wie Stickstoff, Argon, niedermolekularen Alkanen oder Olefinen verdünnt werden.

Das molare Verhältnis zwischen der Hydroxylgruppen enthaltenden Komponente und Alkin bzw. Allen kann zwischen 0,01 und 100 liegen. Bevorzugt wird der Bereich zwischen 0,1 und 5, und besonders bevorzugt wird der Bereich zwischen 0,7 und 1,3 gewählt.

Durch die Reaktionsbedingungen kann die Selektivität der Reaktion bezüglich der Mono- bzw. Diadditionsprodukte gesteuert werden. Niedrige Verhältnisse zwischen der Hydroxylgruppen enthaltenden Komponente und Alkin bzw. Allen sowie hohe Temperaturen und niedrige Partialdrücke der Reaktanden führen zur bevorzugten Bildung der Monoadditionsprodukte während hohe Verhältnisse zwischen der Hydroxylgruppen enthaltenden Komponente und Alkin bzw. Allen sowie niedrige Temperaturen und hohe Partialdrücke der Reaktanden die Bildung der Bisadditionsprodukte begünstigen. Beispielsweise kann aus Propin oder Allen mit Methanol je nach Reaktionsbedingungen selektiv 2-Methoxypropen oder 2,2-Dimethoxypropan gebildet werden.

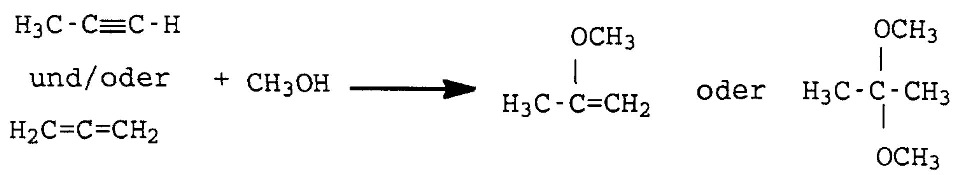

Für die Charakterisierung der Katalysatorproben (frische als auch Ausbauproben) wurden Standardmethoden verwendet. Die gemessene BET-Oberfläche liegt in der Regel zwischen 50 und 500 $m^2/g$. Bevorzugt werden Katalysatoren mit BET-Oberflächen zwischen 80 und 400 $m^2/g$ verwendet. Weiterhin wurden die mit dem neuen Herstellungsverfahren erhaltenen Proben mittels Pulverröntgendiffraktometrie (XRD) untersucht.

Allgemeine Umsetzungsbedingungen

Die katalytischen Umsetzungen gemäß Fig 1. wurden in einem gradientenfreien CSTR (Continuously Stirred Tank Reactor) (A) mit einem Volumen von 1740 ml und einem Katalysatorvolumen von ca. 90 ml, modifiziert für heterogene Gasphasenreaktionen durchgeführt. Der Reaktor hatte einen Innendurchmesser von ca. 108 mm und eine Höhe von ca. 200 mm und wurde mittels einer an der Innenwand angebrachten elektrischen Heizspirale beheizt. In der Mitte des Reaktors war ein kleiner Zylinder aus Metall angebracht (∅ ca. 64 mm, Höhe ca 150 mm), der auf halbe Höhe (ca. 85 mm unterhalb der Oberkante) mit einem Drahtgitter versehen war. Auf dieses Drahtgitter wurde der Katalysator locker aufgeschüttet. Auf dem Deckel des Reaktors war eine flache (∅ ca. 100 mm, Höhe ca. 20 mm) Turbine angebracht, die mit 1500-2000 Upm angetrieben wurde. An der Reaktorachse waren auf verschiedenen Höhen insgesamt 6 Thermoelemente zur Temperaturkontrolle angebracht. Die Edukte wurden unter Druck mittels HPLC-Pumpen dosiert, kurz vor dem Reaktor gemischt und in den Reaktorraum entspannt. Das Alkin bzw. Allen (1 in Fig 1) wurden entweder in reiner Form zudosiert oder als Gemisch mit anderen inerten Komponenten verdünnt. In dem Fall von Propin und Allen wurde ein Gemisch mit anderen Kohlenwasserstoffen eingesetzt (Zusammensetzung: 30-43 Vol% Propin, 16-20 Vol% Allen, 20-45 Vol% Propen, 5-10 Vol% Isobutan und 2-6 Vol% Propan als Hauptkomponenten; alle anderen Komponenten unter 1 %. Dieses Gemisch wurde durch Destillation aus einem Seitenstrom eines Steamcrackers gewonnen). Der Alkoholkomponente (2 in Fig 1) wurden ca. 10 Gew.-% Cyclohexan als interner Standard für die GC-Analytik zudosiert.

Die Reaktion wurde isotherm bei Temperaturen von 120 bis 300°C und einer Zulaufrate von 0,5 bis 10 mmol/min Propin und/oder Allen und 0,5 bis 20 mmol/min MeOH durchgeführt. Der Reaktionsdruck betrug 1,1 bis 3,5 bar (absolut).

Die Gesamtgasmenge, bestehend aus Edukten, Inertgas und internem Standard, betrug in der Regel zwischen 4 und 60 N1/h. Die GHSV (gas hourly space velocity), die definiert ist als

$$GHSV = Gasvolumen\ [N1/h]/Katalysatorvolumen \quad [1]$$

betrug zwischen 80 und 1200 $h^{-1}$. Die LHSV (liquid hourly space velocity), die definiert ist als LHSV = Flüssigkeitsvolumen [N1/h]/Katalysatorvolumen [1] (hier gefördertes Volumen an Propin und MeOH Volumen) betrug zwischen 0,2 und 3 $h^{-1}$. Die Verweilzeit, definiert als Quotient aus dem Katalysatorvolumen [1] und der Gasmenge [N1/s] betrug zwischen 3 und 40 s.

Die Reaktionsgase wurden nach Verlassen des Reaktors über eine beheizte Transferleitung (3) zu einem On-line Gaschromatographen (B) geführt und dort alle 2 h analysiert. Danach wurde der Gasstrom einer partiellen Kondensation (C) unterworfen und der bei Raumtemperatur nicht kondensierbare Anteil (6) wurde in regelmäßigen Abständen (ca. 12 h) mittels Off-line GC analysiert. Das Kondensat (5) wurde ebenfalls gesammelt und mittels Off-line GC analysiert.

Die erfindungsgemäß erhältlichen Enolether der Formel I und die Dialkoxyverbindungen der Formel II sind wertvolle Zwischenprodukte zur Herstellung von Wirkstoffen und Riechstoffen. Insbesondere die Enolether sind begehrte Ausgangsstoffe z.B. zur Herstellung von $\gamma,\delta$-ungesättigten Ketonen als Vorprodukte für die Herstellung von Isophytol.

Will man vor allem die Enolether gewinnen, kann man in an sich bekannter Weise die Verbindungen der Formel II durch Abspaltung von einem Mol $R^1OH$ in die entsprechenden Enolether der Formel I überführen. Dafür existieren zahlreiche aus DE-A- 35 35 128, DE-A- 37 22 891, DE-A-38 04 162, Chemical Abstracts, Vol. 94 (19); 156 241 f und DE-A-19544450 bekannte Verfahren.

Beispiel 1

Herstellung von $Zn_4Si_2O_7(OH)_2 \cdot H_2O$ mit Zn/Si =2 (stöchiometrischer Hemimorphit) und katalytische Umsetzung

In einem 8-1-Rührbehälter stellte man aus 4,5 l vollentsalztem Wasser und 145,1 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 1,5 Mol $SiO_2$ und 0,89 Mol Na her. Ferner wurden 910,7 g $Zn(NO_3)_2 \cdot 6\ H_2O$ (98 %-ig) in 2,25 l vollentsalztem Wasser bei Raumtemperatur gelöst, wobei eine Lösung B mit 3 Mol Zn und 6 Mol $NO_3$ erhalten wurde. Schließlich wurde eine wäßrige Lösung aus 204,4 g NaOH in 225 ml vollentsalztem Wasser hergestellt, wobei eine Lösung C mit einem Na-Gehalt von 5,11 Mol erhalten wurde. Nun wurden die Lösungen B und C bei Raumtemperatur in die Suspension A gegeben, wobei sich eine milchige Suspension D mit folgenden Elementanteilen ergab: Zn-Gehalt = 3 Mol, Si-Gehalt = 1,5 Mol, Na-Gehalt = 6 Mol, $NO_3$-Gehalt = 6 Mol. Der pH-Wert der erhaltenen Suspension D betrug 7,1. Die Suspension D wurde auf 90°C aufgeheizt und bei dieser Temperatur 24 Stunden lang mit einer Umdrehungszahl von 200 Upm gerührt. Die Suspension wurde anschließend auf Raumtemperatur abgekühlt und ein End-pH-Wert von 7,0 gemessen. Der auskristallisierte weiße Niederschlag wurde abfiltriert und mit vollentsalztem Wasser Na-frei gewaschen und der anfallende Filterkuchen bei 90°C in einem Trockenschrank getrocknet. Das getrocknete weiße Pulver wurde röntgenographisch untersucht, wobei sich ein Röntgenpulverdiagramm ergab, das dem der Karteikarte 5-0555 der JCPDS-ICDD-Kartei (1995) voll entsprach und damit die Herstellung von $Zn_4Si_2O_7(OH)_2 \cdot H_2O$ anzeigte. Die nach BET bestimmte spezifische Oberfläche des erhaltenen Pulvers betrug 30 $m^2/g$.

Zur Herstellung eines Katalysators wurde das noch feuchte Pulver direkt zu Strängen verpresst (∅ = 3 mm, Preßdruck = 50 bar), die anschließend 16 h bei 120°C getrocknet wurden. Der fertigen Katalysator hatte eine BET-Oberfläche von 26 $m^2/g$, eine Härte von 6 N/Formkörper.

In die unter (a) beschriebene Apparatur wurden ca. 90 ml des Katalysators eingefüllt und das Propin/Allen-Gemisch (ca. 63 Vol%-ig, 2,8 mmol/min) und Methanol (4,7 mmol/min; Gesamtzulauf mit Inerten: 9,4 mmol/min; Verhältnis MeOH/(Propin+Allen)=1,68) wurden dann mittels HPLC-Pumpen zudosiert. Die Reaktionstemperatur betrug 200°C und der Druck 1,25 bar (Partialdruck der Edukte: 1,0 bar). Es wurden von Anfang an (d.h. der Katalysator hat keine Formierungszeit) folgende Selektivitäten beobachtet: 2-Methoxypropen = 91 %; 2,2-Dimethoxypropan = 5 %; Aceton = 1 %; cis und trans 1-Methoxypropen = 3 %. Der Propin/Allen-Umsatz betrug 48 %. Für den Katalysator wurden nach dem Ausbau die folgenden Werte ermittelt: BET = 25 $m^2/g$, Härte = 6 N/Formkörper.

Beispiel 2

Katalysatorherstellung von Hemimorphit mit Zn/Si = 2,2 und katalytische Umsetzung

In einem 6-l-Rührbehälter wurde aus 3,0 l vollentsalztem Wasser und 96,8 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 1,0 Mol $SiO_2$ und 0,59 Mol Na und aus 667,8 g $Zn(NO_3)_2 \cdot 6\ H_2O$ (98 %-ig) in 1,5 l vollentsalztem Wasser bei Raumtemperatur eine Lösung B mit 2,2 Mol Zn und 4,4 Mol $NO_3$ sowie eine wäßrige Lösung C mit einem Na-Gehalt von 3,81 Mol aus 152,3 g NaOH in 400 ml vollentsalztem Wasser hergestellt. Die Lösungen B und C wurden in die Suspension A bei Raumtemperatur gegeben, wobei sich eine milchige Suspension D mit folgenden Elementanteilen ergab: Zn-Gehalt = 2,2 Mol, Si-Gehalt = 1 Mol, Na-Gehalt = 4,4 Mol, $NO_3^-$-Gehalt = 4,4 Mol. Der pH-Wert der erhaltenen Suspension D betrug 7,2. Die Suspension D wurde auf 90°C erhitzt und bei dieser Temperatur 24 Stunden lang mit einer Umdrehungszahl von 200 Upm gerührt. Nach Abkühlen der Suspension auf Raumtemperatur wurde ein End-pH-Wert von 7,0 gemessen. Der angefallene weiße Niederschlag wurde abfiltriert, mit vollentsalztem Wasser Na-frei gewaschen und der erhaltene Filterkuchen bei 90°C in einem Trockenschrank getrocknet.

Das getrocknete weiße Pulver wurde röntgenographisch untersucht, wobei sich ein Röntgenpulverdiagramm ergab, das dem der Karteikarte 5-0555 der JCPDS-ICDD-Kartei (1995) voll entsprach und damit die Herstellung von $Zn_4Si_2O_7(OH)_2 \cdot H_2O$ anzeigte. Die nach BET bestimmte spezifische Oberfläche des erhaltenen Pulvers betrug 60 $m^2$/g.

650 g des Pulvers wurden mit 20,2 g Magnesiumstearat (Merck) vermischt und zu 20 mm Tabletten gepreßt. Diese Tabletten wurden zu Splitt (<0,5 mm) zerdrückt und der Split zu Ringlochtabletten (5 x 5 x 2 mm) verarbeitet. Die Tabletten wurden dann 10 Stunden bei 350°C kalziniert. Der fertige Katalysator hatte eine BET-Oberfläche von 44 $m^2$/g, und eine Härte von 44 N/Formkörper.

In die unter (a) beschriebene Apparatur wurden ca. 90 ml des Katalysators eingefüllt und das Propin/Allen-Gemisch (ca. 60 Vol%-ig, 2,8 mmol/min) und Methanol (2,0 mmol/min; Gesamtzulauf mit Inerten: 6,8 mmol/min; Verhältnis MeOH/ (Propin+Allen) = 0,72) mittels HPLC-Pumpen zudosiert. Die Reaktionstemperatur betrug 170°C und der Druck 1,1 bar (abs) (Partialdruck der Edukte: 0,8 bar). Es wurden von Anfang an (d.h. der Katalysator hatte keine Formierungszeit) folgende Selektivitäten beobachtet: 2-Methoxypropen = 90 %; 2,2-Dimethoxypropan = 3 %; Aceton = 2 %; cis und trans 1-Methoxypropen = 2 %. Der Propin/Allen-Umsatz betrug 71 % und der Methanol-Umsatz 98 %.

Für den Katalysator wurden nach dem Ausbau die folgenden physikalischen Werte ermittelt: BET 44 = $m^2$/g; Härte = 12 N/Formkörper.

Beispiel 3

Hydrothermale Herstellung von $Zn_4Si_2O_7(OH)_2 \cdot H_2O$ mit Zn/Si = 2 und katalytische Umsetzung

In einem 2-1-Rührbehälter wurde aus 200 ml vollentsalztem Wasser und 19,35 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 0,2 Mol $SiO_2$ und 0,12 Mol Na und aus 121,4 g $Zn(NO_3)_2 \cdot 6\ H_2O$ (98 %-ig) in 200 ml vollentsalztem Wasser bei Raumtemperatur eine Lösung B mit 0,4 Mol Zn und 0,8 Mol $NO_3$ sowie eine wäßrige Lösung C mit einem Na-Gehalt von 0,68 Mol aus 27,2 g NaOH in 100 ml vollentsalztem Wasser hergestellt. Die Lösungen B und C wurden in die Suspension A bei Raumtemperatur gegeben, wobei sich eine milchige Suspension D mit folgenden Elementanteilen ergab: Zn-Gehalt = 0,4 Mol, Si-Gehalt = 0,2 Mol, Na-Gehalt = 0,8 Mol, $NO_3$-Gehalt = 0,8 Mol. Der pH-Wert der erhaltenen Suspension D betrug 7,0. Die Suspension D wurde in einem Autoklaven aus einer Hastelloy-Legierung mit einem Innenvolumen von 1,2 l auf 150°C erhitzt und 6 Stunden lang bei dieser Temperatur mit einer Umdrehungszahl von 200 Upm gerührt, wobei ein Überdruck von 7 bar gemessen wurde. Nach Abkühlen der erhaltenen Suspension auf Raumtemperatur wurde ein End-pH-Wert von 7,2 gemessen. Der auskristallisierte weiße Niederschlag wurde abfiltriert und mit vollentsalztem Wasser Na-frei gewaschen und der anfallende Filterkuchen bei 90°C in einem Trockenschrank getrocknet.

Das Röntgenpulverdiagramm des getrockneten weißen Pulvers entsprach dem der Karteikarte 5-0555 der JCPDS-ICDD-Kartei (1995) und beweist die Herstellung von $Zn_4Si_2O_7(OH)_2$ - $H_2O$. Die nach BET bestimmte spezifische Oberfläche des erhaltenen Pulvers betrug 28,7 $m^2$/g.

150 g Steatitkugeln (∅ = 2 mm) wurden auf einem Drehteller mit 19,2 g einer 1,25 Gew.-%-igen wäßrigen Lösung von Walcocel® (Fa. Wolff, Walsrode), 5,60 g des $Zn_4Si_2O_7(OH)_2 \cdot H_2O$-Pulvers und 8,70 g Zink-Pulver beschichtet. Nach dem Trocknen (4 h bei 120°C) erhielt man einen Schalenkatalysator.

In die unter (a) beschriebenen Apparatur wurden ca. 90 ml des Katalysators eingefüllt und das Propin/Allen-Gemisch (ca. 61 Vol%-ig, 3,0 mmol/min) und Methanol (4,7 mmol/min; Gesamtzulauf mit Inerten: 9,7 mmol/min; Verhältnis MeOH/(Propin + Allen) = 1,59) mittels HPLC-Pumpen zudosiert. Die Reaktionstemperatur betrug 200°C und

der Druck 1,25 bar (abs) (Partialdruck der Edukte: 1,0 bar). Der Propin/Allen-Umsatz war von Anfang an konstant und betrug ca. 17 %. Als Produkte bildeten sich (Selektivitäten in Klammern): 2-Methoxypropen (90 %), 2,2-Dimethoxypropan (3 %) und cis- und trans-1-Methoxypropen (6 %).

Beispiel 4 (Vergleichsbeispiel)

Katalysatorherstellung von $\beta$-$Zn_2SiO_4$ (Zn/Si = 2) und katalytische Umsetzung

100 g des gemäß Beispiel 1 hergestellten Hemimorphits wurden in einem Temperofen 1 Stunde lang bei 700°C erhitzt. Das erhaltene Pulver wurde röntgenographisch untersucht, wobei sich ein Röntgenpulverdiagramm ergab, das dem der Karteikarte 14-0653 der JCPDS-ICDD-Kartei (1995) voll entsprach und damit die phasenreine Bildung von $\beta$-$Zn_2SiO_4$ anzeigte. Die nach BET bestimmte spezifische Oberfläche des erhaltenen weißen Pulvers betrug 25 $m^2$/g.

150 g Steatitkugeln (∅ 2 mm) wurden auf einem Drehteller mit 17,2 g einer 1,25 Gew.-%-igen wäßrigen Lösung von Walocel® (Fa. Wolff, Walsrode), 5,50 g des $\beta$-$Zn_2SiO_4$-Pulvers und 5,50 g Zink- Pulver beschichtet. Nach dem Trocknen (4 h bei 120°C) erhielt man einen Schalenkatalysator.

In die unter (a) beschriebene Apparatur wurden ca. 90 ml des Katalysators eingefüllt und das Propin/Allen-Gemisch (ca. 63 Vol%-ig, 3,1 mmol/min) und Methanol (3,9 mmol/min; Gesamtzulauf mit Inerten: 9,0 mmol/min; Verhältnis MeOH/(Propin + Allen) = 1,25) mittels HPLC-Pumpen zudosiert. Die Reaktionstemperatur betrug 200°C und der Druck 1,2 bar (abs). Der propin/Allen-Umsatz war kleiner 1 % und stieg auch nicht mit der Zeit an.

Beispiel 5 (Vergleichsbeispiel)

Katalysatorherstellung von $\alpha$-$Zn_2SiO_4$(Willemit) und katalytische Umsetzung

100 g des gemäß Beispiel 1 hergestellten Hemimorphits wurden in einem Temperofen 1 Stunde lang bei 800°C erhitzt. Das erhaltene pulver wurde röntgenographisch untersucht, wobei sich ein Röntgenpulverdiagramm ergab, das dem der Karteikarte 37-1485 der JCPDS-ICDD-Kartei (1995) voll entsprach und damit die einphasige Herstellung von $\alpha$-$Zn_2SiO_4$ (Willemit) anzeigte. Die nach BET bestimmte spezifische Oberfläche des erhaltenen weißen Pulvers betrug 18 $m^2$/g.

Das Pulver wurde mit 2 Gew.-% Graphit und 3 Gew.-% Magnesiumstearat gemischt und zu 3 x 5 mm Tabletten gepreßt. Diese Tabletten wiesen eine BET-Oberfläche von 0,6 $m^2$/g auf.

In die unter (a) beschriebene Apparatur wurden ca. 90 ml des Katalysators eingefüllt und das Propin/Allen-Gemisch (ca. 62 Vol%ig, 3,3 mmol/min) und Methanol (4,0 mmol/min; Gesamtzulauf mit Inerten: 9,5 mmol/min; Verhältnis MeOH/(Propin + Allen) = 1,21) mittels HPLC-Pumpen zudosiert. Die Reaktionstemperatur betrug 200°C und der Druck 1,25 bar (abs). Der Propin/Allen-Umsatz war kleiner 1 % und stieg auch nicht mit der Zeit an.

Beispiel 6 (Vergleichsbeispiel)

Katalysatoherstellung von $Zn_3Si_4O_{10}(OH)_2 \cdot 4\ H_2O$ (Sauconit) und katalytischer Umsetzung

In einem 6-1-Rührbehälter wurde aus 3 1 vollentsalztem Wasser und 580,5 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 6 Mol $SiO_2$ und 3,56 Mol Na und aus 607,1 g $Zn(NO_3)_2 \cdot 6\ H_2O$ (98 %-ig) in 1,5 l vollentsalztem Wasser bei Raumtemperatur eine Lösung B mit 2 Mol Zn und 4 Mol $NO_3^-$, sowie eine wäßrige Lösung C mit einem Na-Gehalt von 0,44 Mol aus 17,6 g NaOH in 200 ml vollentsalztem Wasser hergestellt. Die Lösungen B und C wurden in die Suspension A bei Raumtemperatur gegeben, wobei sich eine milchige Suspension D mit folgenden Elementanteilen ergab: Zn-Gehalt = 2 Mol, Si-Gehalt = 6 Mol, Na-Gehalt = 4,0 Mol, $NO_3^-$-Gehalt = 4 Mol. Der pH-Wert der erhaltenen Suspension D betrug 6,8. Die Suspension D wurde auf 90°C erhitzt und bei dieser Temperatur 24 Stunden lang mit einer Umdrehungszahl von 200 Upm gerührt. Nach Abkühlen der Suspension auf Raumtemperatur wurde ein End-pH-Wert von 6,9 gemessen. Der angefallene weiße Niederschlag wurde abfiltriert und mit vollentsalztem Wasser Na-frei gewaschen und der erhaltene Filterkuchen bei 90°C in einem Trockenschrank getrocknet.

Das Röntgenpulverdiagramm des getrockneten Pulvers ergab, das es der Karteikarte 29-1393 der JCPDS-ICDD-Kartei (1995) und damit $Zn_3Si_4O_{10}(OH)_2 \cdot 4\ H_2O$ (Sauconit) entspricht. Die nach BET bestimmte spezifische Oberfläche des erhaltenen weißen Pulvers betrug 216 $m^2$/g.

150 g Steatit-Kugeln (∅ = 2 mm) wurden auf dem Drehteller mit 17,5 g einer 1,25 Gew.-%-igen wäßrigen Lösung von Walocel® (Fa. Wolff, Walsrode) und einer Mischung aus 10,2 g Zink-Pulver und 6,9 g Sauconit beschichtet. Der Katalysator wurde dann 4 h bei 120°C an der Luft getrocknet.

In der oben beschriebenen Apparatur wurden ca. 90 ml des Katalysators eingefüllt. Das Propin/Allen-Gemisch (ca. 63 Vol%-ig, 3,8 mmol/min und Methanol (4.7 mmol/min; Gesamtzulauf mit Inerten: 11,0 mmol/min; Verhältnis MeOH/(Propin + Allen) = 1,25) und Methanol wurden dann mittels HPLC-Pumpen zudosiert. Die Reaktionstemperatur betrug 200°C und der Druck 1,25 bar (abs). Der Propin/Allen-Umsatz war kleiner 1 % und stieg auch nicht mit der Zeit an.

Beispiel 7

Herstellung von röntgenamorphem Zinksilikat mit einem molaren Zn/Si-Verhältnis von 2,1 (entspricht einem 5 %-igen Überschuß bezüglich der Zusammensetzung $Zn_4Si_2O_7(OH_2) \cdot H_2O$)

In einem 6 l-Rührbehälter wurden unter ständigem Rühren (100 Upm) in 3,0 l vollentsalztem und 65°C warmem Wasser 48,37 g pulverförmiges Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) gegeben, wobei eine Suspension A mit 0,5 Mol $SiO_2$ und 0,3 Mol Na erhalten wurde. Ferner wurde eine wäßrige Lösung B aus 72,16 g NaOH (1,8 Mol) in 0,2 l vollentsalztem Wasser hergestellt. Schließlich wurden 318,7 g $Zn(NO_3)_2 \cdot 6\ H_2O$ (98 %ig) in 1,5 l vollentsalztem Wasser gelöst, wobei eine Lösung C mit 1,05 Mol Zn und 2,1 Mol $NO_3^-$ erhalten wurde. Daraufhin wurde die Lösung B in die 65°C warme Suspension A gegeben wurde, wobei nach ca. 5 Minuten eine klare Lösung D erhalten wurde. In die erhaltene Lösung D wurde anschließend die Lösung C gegeben. Dabei entstand eine weiße Suspension E mit einem Zn-Gehalt von 1,05 Mol, einem Si-Gehalt von 0,5 Mol, einem Na-Gehalt von 2,1 Mol und einem $NO_3^-$-Gehalt von 2,1 Mol. Die Suspension E wurde bei 65°C 2 Stunden lang unter Rühren (100 Upm) erhitzt und anschließend auf Raumtemperatur abgekühlt. Es wurde nach Abkühlung ein End-pH-Wert von 6,6 gemessen. Der angefallene weiße Niederschlag wurde abfiltriert, mit vollentsalztem Wasser Na-frei gewaschen und der erhaltene Filterkuchen wurde bei 80°C in einem Trokkenschrank getrocknet.

Das getrocknete weiße Pulver wurde unter Anwendung von Cu-$K\alpha_1$-Strahlung ($\lambda$ = 1,5406 Å) im 2θ-Bereich von 10° bis 90° röntgenographisch untersucht, wobei sich ein Pulver-Röntgenbeugungsdiagramm (die Intensität A der gebeugten Röntgenstrahlung aufgetragen als Funktion des zweifachen Beugungswinkels (2θ)) ergab, das in der Fig. 2 wiedergegeben ist und das die Bildung von überwiegenden Mengen des erfindungsgemäßen röntgenamorphen Zinksilikats neben einer geringen Menge an kristallinem ZnO (Karteikarte 5-0664 der JCPDS-ICDD-Kartei (1995)) anzeigt. Das in Fig. 2 wiedergegebene Pulver-Röntgenbeugungsdiagramm wurde mit Hilfe des Software-Paketes DIFFRAC-AT V 3.2/PROFILE, Pearson-VII-Gestaltsfunktion, Version von 1994 (Fa. Siemens) unter der Randbedingungen einer minimalen mittleren quadratischen Abweichung in eine Superposition von separat aufgelösten Beugungsreflexen entfaltet, wobei zum einen die Reflexe des als Nebenbestandteil vorhandenen ZnO und zum anderen die breiten Reflexe des erfindungsgemäßen röntgenamorphen Zinksilikats berücksichtigt wurden. Die Ausmessung des so entfalteten Pulver-Röntgenbeugungsdiagramms des erfindungsgemäßen röntgenamorphen Zinksilikats führte zu Intensitätsmaxima bei $2\theta = 31° \pm 5°$ und bei $2\theta = 61° \pm 7°$. Die nach BET bestimmte spezifische Oberfläche des erhaltenen Pulvers betrug 124,0 $m^2$/g.

Beispiel 8

Herstellung von röntgenamorphem Zinksilikat mit der Zusammensetzung $Zn_4Si_2O_7(OH)_2 \cdot H_2O$ /Zn/Si = 2,1; entspricht 5 % Zn-Überschuß)

In einem 12-1-Rührbehälter wurden unter ständigem Rühren (100 Upm) in 7,5 l vollentsalztes und 80°C warmes Wasser 120,93 g pulverförmiges Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) gegeben, wobei eine Suspension A mit 1,25 Mol $SiO_2$ und 0,74 Mol Na erhalten wurde. Ferner wurde eine wäßrige Lösung B aus 180,4 g NaOH (4,51 Mol) in 0,5 l vollentsalztem Wasser hergestellt. Schließlich wurden 796,0 g $Zn(NO_3)_2 \cdot 6H_2O$ in 2,5 l vollentsalztem Wasser gelöst, wobei eine Lösung C mit 2,625 Mol Zn und 5,25 Mol $NO_3^-$ erhalten wurde. Daraufhin wurde die Lösung B in die 80°C warme Suspension A gegeben, wobei nach ca. 5 Minuten eine klare Lösung D erhalten wurde. In die erhaltene Lösung D wurde anschließend die Lösung C gegeben. Dabei entstand eine weiße Suspension E mit einem Zn-Gehalt von 2,625 Mol, einem Si-Gehalt von 1,25 Mol, einem Na-Gehalt von 5,25 Mol und einem $NO_3^-$-Gehalt von 5,25 Mol. Die Suspension E wurde bei 80°C 2 Stunden lang unter Rühren (100 Upm) erhitzt und anschließend auf Raumtemperatur abgekühlt. Es wurde nach Abkühlung ein End-pH-Wert von 6,5 gemessen. Der angefallene weiße Niederschlag wurde abfiltriert und mit vollentsalztem Wasser Na-frei gewaschen und der erhaltene Filterkuchen wurde bei 80°C in einem Trockenschrank getrocknet.

Das getrocknete weiße Pulver wurde röntgenographisch untersucht, wobei sich ein Röntgenpulverdiagramm ergab, das dem in der Fig. 2 wiedergegeben entspricht und damit die Herstellung von überwiegenden Mengen an

röntgenamorphen Hemimorphit neben einer geringen Menge an kristallinem ZnO (Karteikarte 5-0664 der JCPDS-ICDD-Kartei (1995)) anzeigte. Die nach BET bestimmte spezifische Oberfläche des erhaltenen Pulvers betrug 102,1 $m^2/g$.

Beispiele 9 bis 11

Katalytische Umsetzung

650 g des nach Beispiel 8 hergestellten amorphen Zinksilikats mit der Zusammensetzung von Hemimorphit wurden mit 20,2 g Zn-Stearat vermischt und zu 20-mm-Tabletten vorkompaktiert, anschließend zu Splitt mit einem Durchmesser von < 0,5 mm zerkleinert und dann zu Tabletten mit dem Format 4,75 x 5,2 mm verformt. Jeweils 100 g Portionen des Katalysators wurde dann bei verschiedenen Temperaturen für 10 Stunden an der Luft kalziniert (siehe Tabelle 1).

In der oben beschriebenen Apparatur wurden ca. 90 ml des Katalysators eingefüllt. Propin/Allen-Gemisch (49,8 Vol.-%ig, 2,7 mmol/min) und Methanol (2,1 mmol/min; Gesamtzulauf mit Inerten: 7,6 mmol/min; Verhältnis MeOH (Propin+Allen) = 0,75) wurden dann mittels HPLC-Pumpen zudosiert. Die Reaktionstemperatur betrug 170°C und der Druck 1,2 bar (abs). Die Ergebnisse sind in Tabelle 1 zusammengefaßt (Abkürzungen: 2MP: 2-Methoxypropen; 22DMP: 2,2-Dimethoxypropan; 1MP: 1-Methoxypropen (cis und trans); 11 DMP: 1,1-Dimethoxypropan):

Tabelle 1

| Beispiel | Kalzinierung | Umsatz | Selektivitäten/% | | | | |
|---|---|---|---|---|---|---|---|
| # | °C | Propin/ Allen | 2MP | 22DMP | Aceton | 1MP | 11DMP |
| 9 | 250 | 27% | 83 | 10 | 1 | 5 | 0,6 |
| 10 | 300 | 48% | 86 | 8 | 1 | 4 | 0,5 |
| 11 | 350 | 46% | 86 | 8 | 1 | 4 | 0,6 |

Diese Katalysatoren zeigen praktisch keine Formierungszeit. Die in Tabelle 1 angegebenen Umsätze und Selektivitäten waren von Anfang an quasi gleichbleibend. Die BET-Oberflächen und Härten der Katalysatoren jeweils nach der Kalzinierung und nach dem Ausbau sind in Tabelle 2 zusammengefaßt.

Tabelle 2

| Beispiel | Kalzinierung | BET ($m^2/g$) | | Härte (N-Formkörper) | |
|---|---|---|---|---|---|
| # | °C | nach Kalz. | nach Ausbau | nach Kalz. | nach Ausbau |
| 9 | 250 | 81 | 79 | 20 | 20 |
| 10 | 300 | 82 | 78 | 26 | 35 |
| 11 | 350 | 82 | 64 | 28 | 36 |

**Patentansprüche**

1. Verfahren zur Herstellung von Verbindungen der Formeln I bzw. II

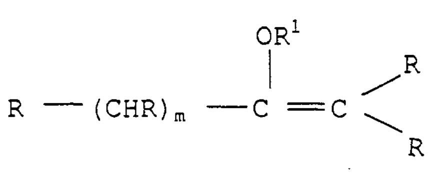

I

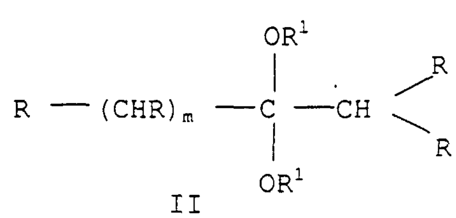

II

in denen $R^1$ Wasserstoff oder einen aliphatischen, cycloaliphatischen, araliphatischen, aromatischen oder heterocyclischen Rest oder einen Acylrest bedeutet, wobei diese Reste weitere Substituenten, die nicht mit Acetylenen oder Allenen reagieren, tragen können und die Reste R unabhängig voneinander für Wasserstoff oder aliphatische, cycloaliphatische, araliphatische, aromatische oder heterocyclische Reste stehen, die unter Bildung eines Ringes

miteinander verbunden sein können und m für 0 oder 1 steht, durch Addition von Verbindungen der Formel III

$$R^1OH \qquad III$$

an Acetylene oder Allene der Formeln IV bzw. V

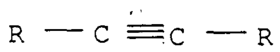

IV

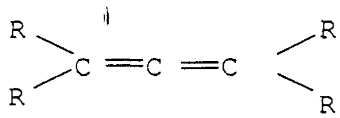

V

wobei $R^1$ und R die oben angegebenen Bedeutungen haben, in der Gasphase bei erhöhter Temperatur in Gegenwart eines heterogenen, ein Silikat enthaltenden Katalysators, **dadurch gekennzeichnet, daß** man einen Katalysator verwendet, der als aktiven Bestandteil ein durch Ausfällung in wäßriger Lösung aus einer löslichen Silizium- und Zinkverbindung erhältliches Zinksilikat enthält oder aus diesem besteht, wobei das Zinksilikat

a) ein im wesentlichen röntgenamorphes Zinksilikat der Formel VI

$$Zn_aSi_cO_{a+2c-0,5e}(OH)_e.fH_2O \qquad VI,$$

ist, in der e die Werte 0 bis zur Summe aus $2_a$ $+4_c$ bedeutet und das Verhältnis a/c 1 bis 3,5 und f/a 0 bis 200 beträgt und
b) das Zinksilikat durch Ausfällung aus wäßriger Lösung aus einem Alkalisilikat und einem Zinksalz bei einem pH-Wert von 4 bis 9,5 erhalten worden ist.

**2.** Verfahren gemäß Anspruch 1, **dadurch gekennzeichnet, daß** das Zinksilikat röntgenamorph ist und eine atomare Raumordnung hat, die unter Anwendung von Cu-$Ka_1$-Strahlung (1=1,5406 Å) ein Pulver-Röntgenbeugungsdiagramm (die Intensität A der gebeugten Röntgenstrahlung aufgetragen als Funktion des zweifachen Beugungswinkels (2q)) bedingt, das im 2q-Bereich von 10° bis 90° breite Intensitätsmaxima bei 31° ± 5° und bei 61° ± 7° aufweist.

**3.** Verfahren gemäß Anspruch 1, **dadurch gekennzeichnet, daß** man einen Katalysator verwendet, dessen katalytisch wirksame Komponente mit bis zu 80 Mol-% noch mit weiteren Metallen dotiert ist, ausgewählt aus
der Gruppe A, bestehend aus Beryllium, Magnesium, Calcium, Strontium, Barium. Mangan, Eisen, Kobalt, Nickel, Kupfer; Cadmium und Quecksilber, und
der Gruppe B, bestehend aus Titan, Zirkon, Hafnium, Germanium, Zinn und Blei,
wobei in der Formel VI die Elemente der Gruppe A teilweise Zink und die Elemente der Gruppe B teilweise Silizium ersetzen.

**4.** Verfahren gemäß Anspruch 1, **dadurch gekennzeichnet, daß** die Addition der Hydroxylgruppen enthaltenden Verbindung $R^1OH$ an die Verbindungen der Formeln IV und V bei Temperaturen von 100°C bis 350°C durchführt.

**5.** Verfahren gemäß Anspruch 4, **dadurch gekennzeichnet, daß** man bei der Umsetzung weitere Salze von Elementen
der Gruppe A, bestehend aus Beryllium, Magnesium, Calcium, Strontium, Barium, Mangan, Eisen, Kobalt, Nickel, Kupfer, Cadmium und Quecksilber, und
der Gruppe B, bestehend aus Titan, Zirkon, Hafnium, Germanium, Zinn und Blei,
in Mengen von 0 bis 20 Mol-% der Gruppe A, bezogen auf Zink, und 0 bis 20 Mol-% der Gruppe B, bezogen auf Silicium, mitverwendet.

**6.** Verfahren gemäß Anspruch 1, **dadurch gekennzeichnet, daß** man 2-Methoxypropen und/oder 2,2-Dimethoxypropan durch Addition von Methanol an Methylacetylen und/oder Allen bei Temperaturen von 100°C bis 350°C

herstellt.

**7.** Verfahren zur Herstellung eines für das Verfahren gemäß Anspruch 1 geeigneten röntgenamorphen Zinksilikats, **dadurch gekennzeichnet, daß** man eine wäßrige Suspension eines Alkalisilikats mit einer wäßrigen Lösung eines Zinksalzes bei

a) Temperaturen von 20°C bis zum Siedepunkt der sich ergebenden wäßrigen Suspension bei
b) einem pH-Wert von 4 bis 9,5,
c) in solchen Mengenverhältnissen von Alkalisilikat und Zinksalz umsetzt, daß die Bedingungen der Formel VI erfüllt werden und
d) eine solche Verweilzeit einhält, daß noch nicht in erheblichem Maße Kristallisation des Zinksilikats eintritt.

**8.** Röntgenamorphes Zinksilikat, **gekennzeichnet**

a) durch die Zusammensetzung entsprechend der Formel VI

$$Zn_aSi_cO_{a+2c-0,5e}(OH)_e.fH_2O \qquad VI,$$

in der e die Werte 0 bis zur Summe aus $2_a$ $+4_c$ und das Verhältnis a:c 1 bis 3,5 und f:a 0 bis 25 beträgt und
b) daß es durch Ausfällung aus wäßriger Lösung aus einem Alkalisilikat und einem Zinksalz bei einem pH-Wert von 4 bis 9,5 erhalten worden ist.

**9.** Röntgenamorphes Zinksilikat nach Anspruch 8 **dadurch gekennzeichnet, daß** es eine atomare Raumordnung hat, die unter Anwendung von Cu-$Ka_1$-Strahlung (1 = 1,5406 Å) ein Pulver-Röntgenbeugungsdiagramm (die Intensität A der gebeugten Röntgenstrahlung aufgetragen als Funktion des zweifachen Beugungswinkels (2q)) bedingt, das im 2q-Bereich von 10° bis 90° breite Intensitätsmaxima bei 31° ± 5° und bei 61° ± 7° aufweist.

**10.** Röntgenamorphes Zinksilikat gemäß Anspruch 9, **dadurch gekennzeichnet, daß** das Molverhältnis Zink zu Silizium 1,9 bis 2,3 beträgt.

**11.** Verwendung eines röntgenamorphen Zinksilikats gemäß Anspruch 9 als Katalysator zur Addition von Hydroxylgruppen enthaltenden Verbindungen an Acetylene oder Allene.

## Claims

**1.** A process for the preparation of compounds of the formulae I and II

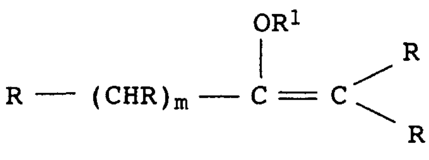

I

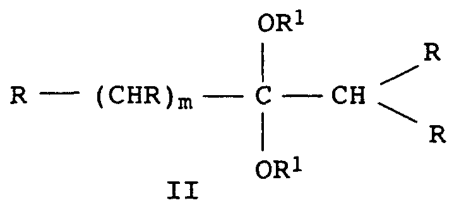

II

where $R^1$ is hydrogen or an aliphatic, cycloaliphatic, araliphatic, aromatic or heterocyclic radical or an acyl radical, it being possible for these radicals to carry further substituents which do not react with acetylenes or allenes, the radicals R, independently of one another, are hydrogen or aliphatic, cycloaliphatic, araliphatic, aromatic or heterocyclic radicals which may be bonded to one another with formation of a ring, and m is 0 or 1, by an addition reaction of a compound of the formula III

$$R^1OH \qquad III$$

with an acetylene or allene of the formula IV or V

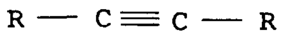

IV

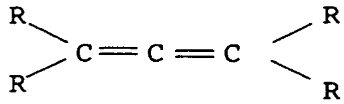

V

where $R^1$ and R have the abovementioned meanings, in the gas phase at elevated temperatures in the presence of a heterogeneous, silicate-containing catalyst, wherein the catalyst used is one which contains or consists of, as the active component, a zinc silicate obtainable by precipitation in aqueous solution from a soluble silicon compound and zinc compound, zinc silicate being

a) an essentially X-ray amorphous zinc silicate of the formula VI

$$Zn_aSi_cO_{a+2c-0.5e}(OH)_e.fH_2O \quad VI,$$

where e is from 0 to 2a + 4c and the ratio a:c is from 1 to 3.5, and the ratio f:a is from 0 to 200, and
b) the zinc silicate having been obtained by precipitation from an aqueous solution of an alkali metal silicate and a zinc salt at a pH of from 4 to 9.5.

**2.** A process as claimed in claim 1, wherein the zinc silicate is X-ray amorphous and has a three-dimensional atomic structure which, using Cu-K$\alpha_1$ radiation ($\lambda$ = 1.5406 Å), gives a powder X-ray diffraction pattern (the intensity A of the diffracted X-ray is plotted as a function of twice the diffraction angle (2$\theta$)) which, in the 2$\theta$ range from 10° to 90°, has broad intensity maxima at 31° ± 5° and at 61° ± 7°.

**3.** A process as claimed in claim 1, wherein the catalyst used is one whose catalytically active component is furthermore doped with up to 80 mol% of further metals selected from
the group A consisting of beryllium, magnesium, calcium, strontium, barium, manganese, iron, cobalt, nickel, copper, cadmium and mercury and from
the group B consisting of titanium, zirconium, hafnium, germanium, tin and lead,
the elements of group A replacing some of the zinc and the elements of group B replacing some of the silicon in the formula VI.

**4.** A process as claimed in claim 1, wherein the addition reaction of the hydroxyl-containing compound $R^1OH$ with the compounds of the formulae IV and V is carried out at from 100 to 350°C.

**5.** A process as claimed in claim 4, wherein further salts of elements
of group A consisting of beryllium, magnesium, calcium, strontium, barium, manganese, iron, cobalt, nickel, copper, cadmium and mercury and
of group B consisting of titanium, zirconium, hafnium, germanium, tin and lead
are present during the reaction in amounts of from 0 to 20 mol%, based on zinc, of group A and from 0 to 20 mol%, based on silicon, of group B.

**6.** A process as claimed in claim 1, wherein 2-methoxypropene and/or 2,2-dimethoxypropane is prepared by an addition reaction of methanol with methylacetylene or allene at from 100 to 350°C.

**7.** A process for the preparation of an X-ray amorphous zinc silicate suitable for the process as claimed in claim 1, wherein an aqueous suspension of an alkali metal silicate is reacted with an aqueous solution of a zinc salt at

a) from 20°C to the boiling point of the resulting aqueous suspension, at
b) a pH of from 4 to 9.5, and
c) in ratios of alkali metal silicate to zinc salt such that the conditions of the formula VI are fulfilled, and
d) a residence time is maintained such that considerable crystallization of the zinc silicate does not as yet occur.

**8.** An X-ray amorphous zinc silicate, comprising

a) the composition corresponding to the formula VI

$$Zn_aSi_cO_{a+2c-0.5e}(OH)_e \cdot fH_2O \qquad VI,$$

where e is from 0 to 2a + 4c and the ratio a:c is from 1 to 3.5 and the ratio f:a is from 0 to 25 and wherein
b) said zinc silicate has been obtained by precipitation from an aqueous solution of an alkali metal silicate and a zinc salt at a pH of from 4 to 9.5.

**9.** An X-ray amorphous zinc silicate as claimed in claim 8, wherein said zinc silicate has a three-dimensional atomic structure which, using Cu-K$\alpha_1$ radiation ($\lambda$ = 1.5406 Å), gives a powder X-ray diffraction pattern (the intensity A of the diffracted X-ray is plotted as a function of twice the diffraction angle (2θ)) which, in the 2θ range of from 10° to 90°, has broad intensity maxima at 31° ± 5° and at 61° ± 7°.

**10.** An X-ray amorphous zinc silicate as claimed in claim 9, wherein the molar ratio of zinc to silicon is from 1.9 to 2.3.

**11.** The use of an X-ray amorphous zinc silicate as claimed in claim 9 as a catalyst for the addition reaction of hydroxyl-containing compounds with acetylenes or allenes.

**Revendications**

**1.** Procédé de fabrication de composés de formule I et II

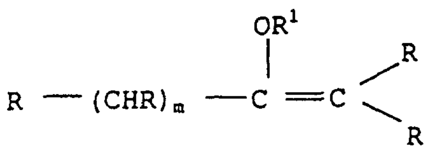

I

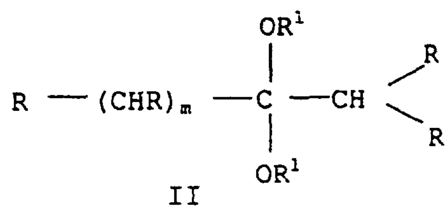

II

dans lesquelles $R^1$ représente un atome d'hydrogène ou un résidu aliphatique, cycloaliphatique, araliphatique, aromatique ou hétérocyclique ou un résidu acyle, où ces résidus possèdent éventuellement d'autres substituants qui ne réagissent pas avec les acétylènes ou les allènes, et les restes R représentent indépendamment les uns des autres un atome d'hydrogène ou un résidu aliphatique, cycloaliphatique, araliphatique, aromatique ou hétérocyclique, résidus qui peuvent être liés entre eux pour former un cycle, et m représente le nombre 0 ou 1, par le moyen d'une addition de composés de formule III

$$R^1OH \qquad III$$

aux acétylènes ou aux allènes, de formule IV respectivement V

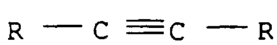

IV

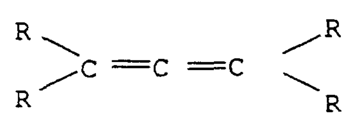

V

où $R^1$ et R prennent les définitions énoncées ci-dessus, en phase gazeuse, à une température élevée, en présence

d'un catalyseur hétérogène contenant un silicate, **caractérisé en ce que** l'on met en oeuvre un catalyseur contenant, en tant que composant actif, un silicate de zinc que l'on peut obtenir, par le moyen d'une précipitation en solution aqueuse, à partir d'un composé soluble contenant du silicium et du zinc, ou bien un catalyseur constitué par ce silicate de zinc, où le silicate de zinc

a) est un silicate de zinc, essentiellement d'apparence amorphe aux rayons X, de formule VI

$$Zn_aSi_cO_{a+2c-0,5e}(OH)_e.fH_2O \qquad VI,$$

dans laquelle e représente un nombre compris dans la plage de 0 à la somme de $2_a+4_c$, et le rapport a/c est compris dans la plage de 1 à 3,5 et f/a est compris dans la plage de 0 à 200, et
b) a été obtenu par le moyen d'une précipitation en solution aqueuse, à un pH de 4 à 9,5, à partir d'un silicate alcalin et d'un sel de zinc.

**2.** Procédé selon la revendication 1, **caractérisé en ce que** le silicate de zinc est d'apparence amorphe aux rayons X et qu'il présente une configuration spatiale atomique qui, avec la raie Cu-$Ka_1$ ($\lambda$ = 1,5406 Å) produit un diagramme de diffraction des rayons X selon le protocole des poudres (l'intensité A de la diffraction des rayons X est portée en fonction de l'angle double de diffraction (2q)) qui présente, dans le secteur 2q dans la plage de 10° à 90°, de larges maximums d'intensité à 31° ± 5° et à 61° ± 7°.

**3.** Procédé selon la revendication 1, **caractérisé en ce que** l'on met en oeuvre un catalyseur dont le composant à action catalytique est dopé, jusqu'à 80% en moles, d'autres métaux choisis dans

- le groupe A formé par le béryllium, le magnésium, le calcium, le strontium, le baryum, le manganèse, le fer, le cobalt, le nickel, le cuivre, le cadmium et le mercure, et
- le groupe B formé par le titane, le zirconium, l'hafnium, le germanium, l'étain et le plomb,

où, dans la formule VI, les éléments du groupe A remplacent en partie le zinc et les éléments du groupe B remplacent en partie le silicium.

**4.** Procédé selon la revendication 1, **caractérisé en ce que** l'on ajoute le composé $R^1OH$ comprenant des groupes hydroxyle, aux composés de formule IV et V, à une température comprise dans la plage de 100°C à 350°C.

**5.** Procédé selon la revendication 4, **caractérisé en ce que**, pour la réaction, on met en oeuvre également d'autres sels d'éléments

- du groupe A formé par le béryllium, le magnésium, le calcium, le strontium, le baryum, le manganèse, le fer, le cobalt, le nickel, le cuivre, le cadmium et le mercure, et
- du groupe B formé par le titane, le zirconium, l'hafnium, le germanium, l'étain et le plomb,

en quantité comprise dans la plage de 0 à 20% en moles du groupe A par rapport au zinc, et comprise dans la plage de 0 à 20% en moles du groupe B par rapport au silicium.

**6.** Procédé selon la revendication 1, **caractérisé en ce que** l'on prépare le 2-méthoxypropène et/ou le 2,2-diméthoxypropane au moyen d'une addition du méthanol au méthylacétylène et/ou à l'allène, à une température comprise dans la plage de 100°C à 350°C.

**7.** Procédé pour la préparation d'un silicate de zinc qui est d'apparence amorphe aux rayons X et qui est approprié selon la revendication 1, **caractérisé en ce que** l'on fait réagir une suspension d'un silicate alcalin aqueuse avec une solution d'un sel de zinc aqueuse

a) à une température comprise entre 20°C et le point d'ébullition de la suspension aqueuse résultante
b) à un pH compris dans la plage de 4 à 9,5,
c) de sorte que, en ce qui concerne le rapport de la quantité de silicate alcalin au sel de zinc, les conditions de la formule VI sont respectées, et
d) que l'on choisit le temps de repos de telle sorte que la cristallisation du silicate de zinc n'ait pas encore

démarré dans une grande mesure.

**8.** Silicate de zinc qui est d'apparence amorphe aux rayons X, **caractérisé en ce que**

a) la constitution respecte la formule VI

$$Zn_aSi_cO_{a+2c-0,5e}(OH)_e.fH_2O \qquad VI,$$

dans laquelle e représente un nombre compris dans la plage de 0 à la somme de $2_a+4_c$, et le rapport a/c est compris dans la plage de 1 à 3,5 et f/a est compris dans la plage de 0 à 25, et

b) on l'a obtenu au moyen d'une précipitation en solution aqueuse, à un pH de 4 à 9,5, à partir d'un silicate alcalin et d'un sel de zinc.

**9.** Silicate de zinc d'apparence amorphe aux rayons X selon la revendication 8, **caractérisé en ce qu'**il présente une configuration spatiale atomique qui, avec la raie Cu-$Ka_1$ ($\lambda$ = 1,5406 Å) produit un diagramme de diffraction des rayons X selon le protocole des poudres (l'intensité A de la diffraction des rayons X est portée en fonction de l'angle double de diffraction (2q)) qui présente, dans le secteur 2q compris dans la plage de 10° à 90°, de larges maximums d'intensité à 31° ± 5° et à 61° ± 7°.

**10.** Silicate de zinc d'apparence amorphe aux rayons X selon la revendication 9, **caractérisé en ce que** le rapport molaire de zinc au silicium est compris dans la plage de 1,9 à 2,3.

**11.** Mise en oeuvre d'un silicate de zinc d'apparence amorphe aux rayons X selon la revendication 9 en tant que catalyseur pour l'addition de composés comprenant des groupes hydroxyle, aux acétylènes ou aux allènes.

FIG.1

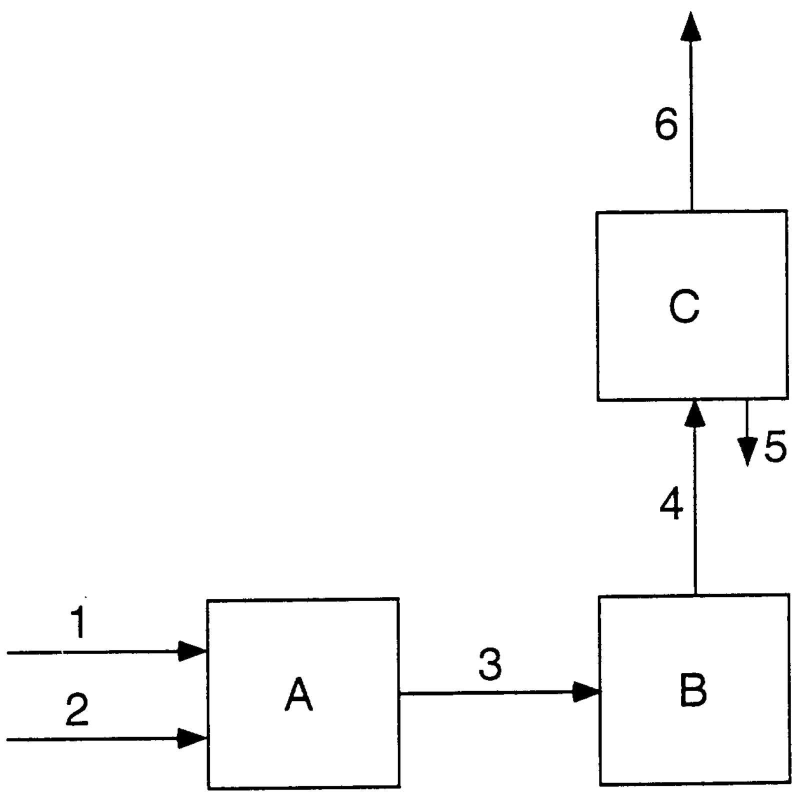

FIG.2